# EUROPEAN PATENT SPECIFICATION

(11) **EP 1 435 964 B1**
(45) Date of publication and mention of the grant of the patent: **09.12.2009**
(21) Application number: 02773763.4
(22) Date of filing: 16.10.2002
(51) Int. Cl.: A61K 31/55, A61K 31/535, A61K 31/40, A61K 31/335, A61K 31/235, A61K 31/24, A61K 31/195, C07D 209/48, C07D 223/18, C07D 305/14, C07D 323/00, C07D 413/00

(54) **NOVEL PRODRUGS OF N-H BOND-CONTAINING COMPOUNDS AND METHODS OF MAKING THEREOF**
NEUE PRODRUGS VON N-H-BINDUNG ENTHALTENDEN VERBINDUNGEN UND VERFAHREN ZU IHRER HERSTELLUNG
NOUVEAUX PROMEDICAMENTS DE COMPOSES CONTENANT DES LIAISONS N-H ET LEURS PROCEDES DE PREPARATION

(30) Priority: 16.10.2001 US 329868 P
(43) Date of publication of application: 14.07.2004
(73) Proprietor: THE UNIVERSITY OF KANSAS, Lawrence, KS 66045-7563 (US)
(72) Inventor: GUARINO, Victor, R., Lawrence, KS 66049 (US); KARUNARATNE, Veranja, Lawrence, KS 66049 (US); STELLA, Valentino, J., Lawrence, KS 66044 (US)
(74) Representative: Thomson, Craig Richard
(86) International application number: PCT/US2002/032957
(87) International publication number: WO 2003/032908

(56) References cited:
- EP-A- 0 065 658
- EP-A- 0 141 119
- WO-A-98/08833
- DE-A- 2 135 543
- DE-A- 2 212 692
- US-A- 3 980 787
- T.G. BACK ET AL: J. ORG. CHEM., vol. 54, no. 8, 1989, pages 1904-1910, XP002303955
- DATABASE CAPLUS CHEMICAL ABSTRACTS SERVICE, COLUMBUS, OHIO, US; XP002303945 retrieved from STN Database accession no. 1963:461857 & JP 38 001630 A (CHUGAI PHARMACEUTICAL CO LTD) 1 March 1963 (1963-03-01)
- M.J. GIL ET AL: BIOORG. MED. CHEM. LETT., vol. 9, 1999, pages 2321-2324, XP004174183
- DATABASE CAPLUS CHEMICAL ABSTRACTS SERVICE, COLUMBUS, OHIO, US; XP002303944 retrieved from STN Database accession no. 1958:55602 & M. PROTIVA ET AL: CESKO-SLOVENSKA FARMACIE, vol. 6, 1957, pages 425-431,
- DATABASE CAPLUS CHEMICAL ABSTRACTS SERVICE, COLUMBUS, OHIO, US; XP002306041 retrieved from STN Database accession no. 1980:146617 -& JP 54 128578 A (SANKYO CO LTD) 5 October 1979 (1979-10-05)
- DATABASE CAPLUS CHEMICAL ABSTRACTS SERVICE, COLUMBUS, OHIO, US; XP002306043 retrieved from STN Database accession no. 1963:455473 & G. FENECH ET AL: ANNALI DI CHIMICA, vol. 53, no. 6, 1968, pages 873-882,
- DATABASE CAPLUS CHEMICAL ABSTRACTS SERVICE, COLUMBUS, OHIO, US; XP002306042 retrieved from STN Database accession no. 2001:56879 -& JP 2001 019684 A (AGENCY OF INDUSTRIAL SCIENCES AND TECHNOLOGY) 23 January 2001 (2001-01-23)
- CAPOZZI ET AL.: 'Ortho-thioquinones, new acceptors for the stereoselective synthesis of aryl 2-deoxy-O-glycosides' CHEMISTRY: A EUROPEAN JOURNAL vol. 5, no. 6, June 1999, pages 1748 - 1754, XP002963602

## Description

### FIELD OF THE INVENTION

The present invention relates to novel prodrugs of pharmaceutical compounds containing one or more N-H bonds. More specifically, the present embodiment of the invention relates to prodrugs wherein sulfur-containing promoieties are attached to pharmaceutical compounds which contain one or more N-H bonds to produce prodrugs containing at least one N-S bond. These N-S bond-containing prodrugs could have optimized stability, solubility, cell membrane permeability, pharmacokinetic properties and other pharmaceutical properties over the pharmaceutical compounds from which they are formed, depending upon the nature of the promoiety. Reversion of the prodrug to the parent pharmaceutical compound occurs by the reaction of the prodrugs with thiol molecules such as cysteine, glutathione or any other thiol containing molecule. Further, the present invention relates to methods of making N-S bond-containing prodrugs of pharmaceutical compounds containing one or more N-H bonds whereby sulfur-containing promoieties are attached to the parent compounds to create at least one N-S bond.

### BACKGROUND OF THE INVENTION

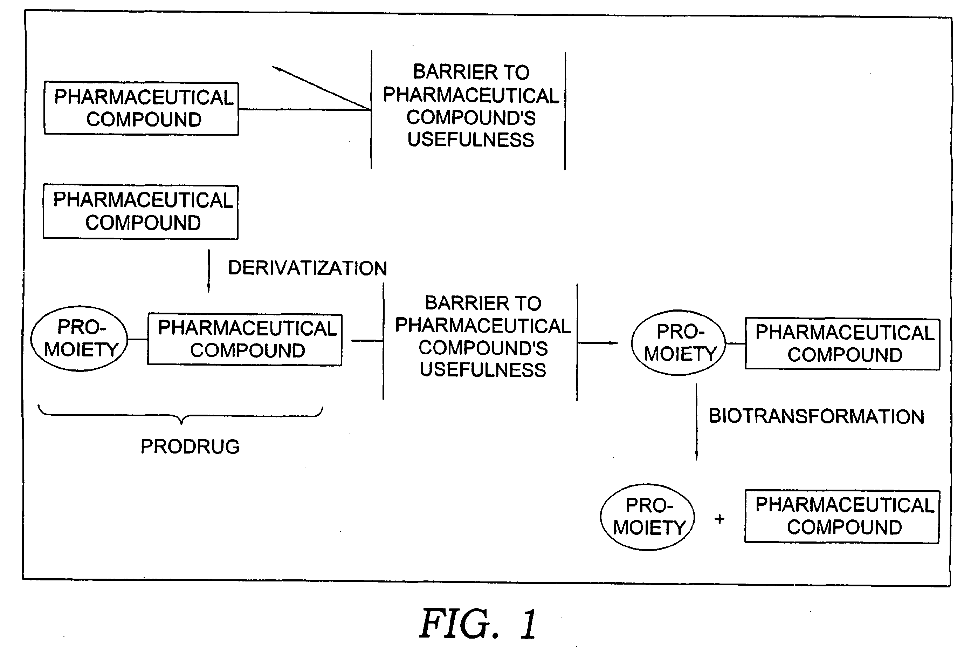

Many pharmaceutical compounds have physicochemical properties which create barriers to attaining their maximum therapeutic potential. Under specific conditions, such compounds may be reversibly modified to overcome their undesirable properties through the creation of prodrugs. The basic concept underlying prodrug chemistry is schematically illustrated in FIG. 1. A compound's physicochemical properties may be temporarily modified by attaching a promoiety to the compound. The compound-promoiety derivative is referred to as a prodrug. In this chemically modified form of the compound, the prodrug is able to overcome the original barrier. Once the barrier is overcome, the prodrug reverts to the parent compound through a process referred to as transformation or reversion. The pharmaceutical compound is then able to interact with its appropriate receptor and elicit the intended pharmacological response at the compound's site of action.

To date, there does not appear to be any known art using technology to create N-S bond-containing prodrugs. However, Bridges studied the inactivation of toxin and carcinogen, N-trichloromethylthio-4-cyclohexane 1,2-dicarboximide or alternatively N-(trichloromethylthio)-3a,4,7,7a-tetrahydrophthalimide ("Captan"), a fungicide used for agricultural purposes and in paints, soaps, paper and leather. Captan has very low solubility and an N-S bond. Captan was administered to rabbits and the levels of Captan were tested. The study found that Captan, in its active form, was broken down in the body due to high thiols in the blood. Thus, this resulted in the rapid inactivation of Captan, thus, lowering the biological and genetic damage caused to a mammalian body by Captan. Bridges, B.A., The Mutagency of Captan and Related Fungicides. Mutation Research, 1975 32: p. 3-34.

Pharmaceutical compounds having certain functional groups which contain one or more N-H bonds, tend to be poorly water- and lipid- soluble due to their hydrogen bond forming potential. Examples of such N-H containing functional groups include, but are not limited to, imides, hydantoins, uracils, barbitals, amides, peptides, ureas, carbamates, aliphatic amines, aromatic amines, sulfonamides and N-containing heterocycles such as imidazole and pyroles. In addition to poor solubility, these functional groups also may lead to permeability problems and other poor pharmacokinetic and pharmaceutical properties including high polarity. Further, the amide and peptide bonds present in some of these functionalities are susceptible to peptidase cleavage. As a result, pharmaceutical Compounds containing these functional groups, while having desirable therapeutic potential, have various barriers to achieving their full potential. Accordingly, for compounds having N-H bond-containing functionalities to achieve maximum therapeutic potential, these barriers must be overcome.

The following publications list some known sulfenyl compounds:
Databse Caplus Chemical Abstracts Service, Columbus, Ohio, US; XP002303945 retrieved from STN Database accession no. 1963:461857;
M.J. Gil et al: Bioorg. Med. Chem. Lett., vol 9, 1999, pages 2321-2324, XP004174183;
WO 98/08833 A (Bristol-Myers Squibb Co) 5 March 1998.

However, none of these documents show N-S bond-containing prodrugs.

### SUMMARY OF THE INVENTION

The present invention is directed to sulfenamide prodrugs of N-H bond-containing compounds, the prodrugs having a general structure as illustrated in the following Formula I, 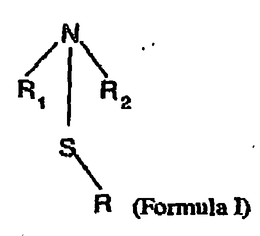
wherein R, R₁ and R₂ are as defined in Claim 1, and pharmaceutically acceptable salts thereof.
R may also include, but is not limited to: 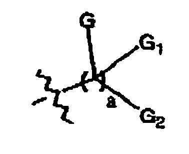
where a is an integer from 0-10 and G, G₁, and G₂, may be the same or different and are: 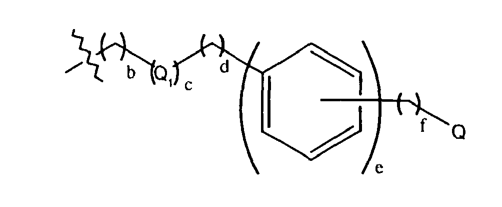
where b, c, d, e and f are integers from 0-10, Q1 is oxygen or sulfur and Q is selected from the group comprising: 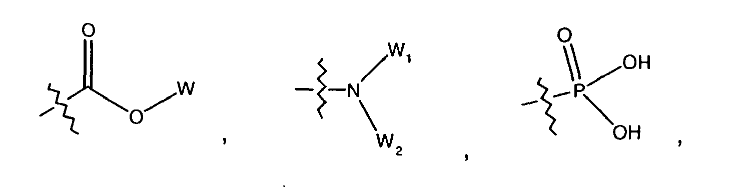 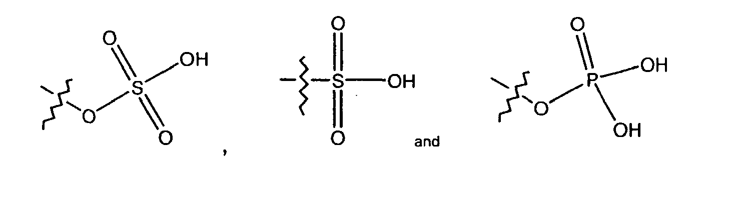
wherein W, W1 and W2 each have the same definition as the organic residue of R as defined in claim 1.

Examples of R may include, but are not limited to: 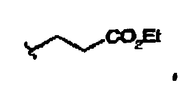 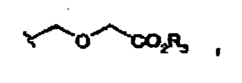 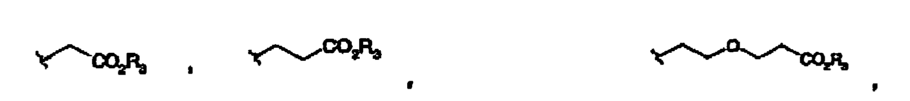 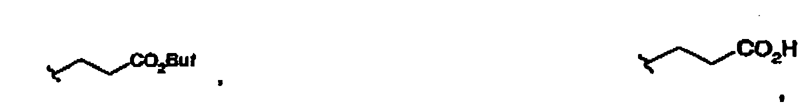 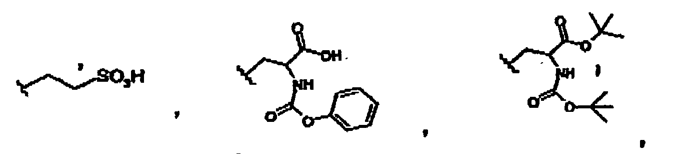 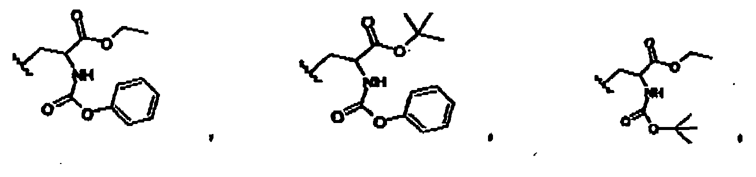 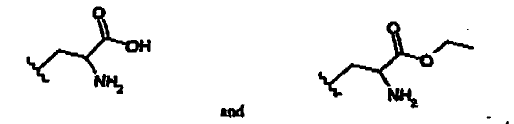 wherein R₃ has the same definition as R as defined in Claim 1.

The compound of formula I may be: 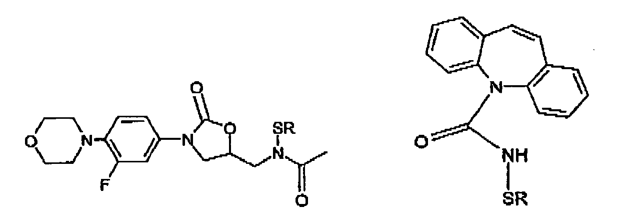 or 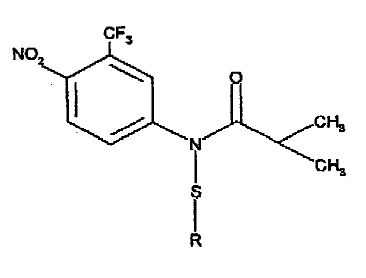 and pharmaceutically acceptable salts thereof, such as 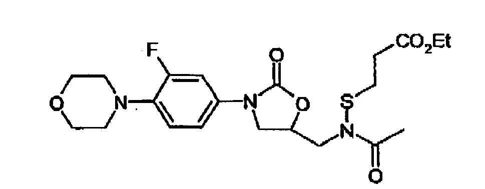 or

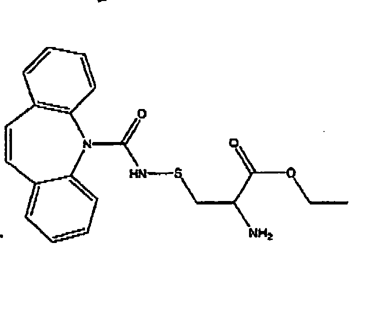

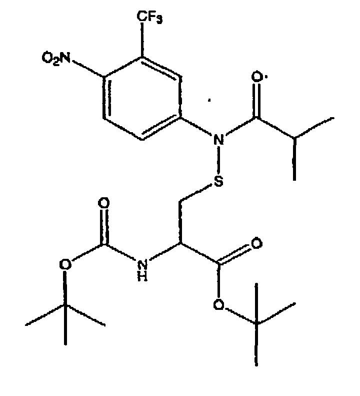

The compound of formula I may also be: 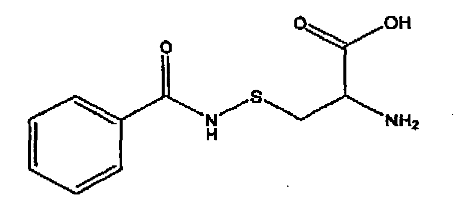 and pharmaceutically acceptable salts thereof.

In another of its aspects, the present invention is directed to a prodrug being a compound as defined in Claim 1 for use in medicine.

The present invention is also directed to a pharmaceutical composition comprising a prodrug as defined in Claim 1 and a pharmaceutically acceptable carrier, preferably further comprising an antimicrobial agent, a preservative, a solubilizer, or a stabilizer.

The prodrug may be: 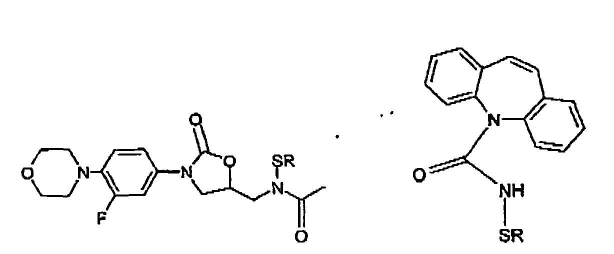 or 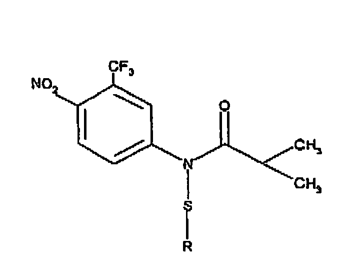 and pharmaceutically acceptable salts thereof.

The present invention is further drawn to methods of making N-S bond-containing prodrugs. One method for the synthesis of prodrugs involves a derivatizing reagent of the general form represented in Formula II,

R-S-H (Formula II)

its salt represented by the general Formula III,

R-S⁻M⁺ (Formula III)

or its disulfide represented by the general Formula IV,

R-S-S-R (Formula IV)

wherein in each of Formulas II, III and IV, R may be polar or non-polar depending upon the changes desired in the properties of the parent pharmaceutical compound. R may be hydrogen or any inorganic or organic residue as described above.

Methods for the synthesis of prodrugs may alternatively involve a derivatizing reagent of the general form represented in Formula V,

R-S-X (Formula V)

wherein X may be any good leaving group including, but not limited to, halides such as chlorine or bromine. R may be hydrogen or an organic or inorganic residue and may be either polar or non-polar, as described above, depending upon the desired changes in the properties of the parent pharmaceutical compound.

Still further, the present invention is directed to a method of making novel prodrugs of N-H bond-containing compounds, the prodrug synthesis illustrated in Reaction Scheme IV, 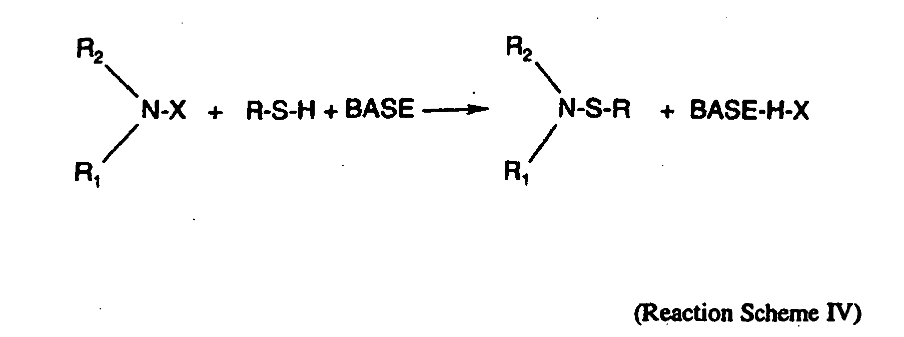 wherein X is any good leaving group such as, but not limited to, halides like chlorine or bromine. It will be understood and appreciated that the illustrated N-X bond-containing compound may be synthesized from an N-H bond-containing compound by any means known to those of skill in the art.

In Reaction Scheme IV, R₁ and R₂ are residues of an N-H containing pharmaceutical compound. R is defined in claim 1, R, R₁ and R₂ may be the same or different and each may be hydrogen or any organic or inorganic residue, as described above.

Further discussion of the synthesis of sulfenamides may be found in Craine, Leslie et, al, The Chemistry of Sulfenamides, Chemical Reviews June 1989: Volume 89, Number 4 and Koval, LV., Synthesis and Application of Sulfenamides, Russian Chemical Reviews 65 (5) 421-440 (1996) and references therein.

The present invention is directed to methods of using novel N-S prodrugs to optimize chemical stability, solubility, cell membrane permeability, pharmacokinetic properties and other pharmaceutical properties over the parent N-H bond-containing pharmaceutical compounds.

Additional aspects of invention, together with the advantages and novel features appurtenant thereto, will be set forth in part in the description which follows, and in part will become apparent to those skilled in the art upon examination of the following, or may be learned from the practice of the invention. The objects and advantages of the invention may be realized and attained by means, instrumentalities and combinations particularly pointed out in the appended claims.

### BRIEF DESCRIPTION OF THE DRAWINGS

In the accompanying drawing which forms a part of the specification and is to be read in conjunction therewith:
FIG. 1 is a schematic representation of the prodrug concept.

### DETAILED DESCRIPTION OF THE INVENTION

The present invention is directed to novel prodrugs of various pharmaceutical compounds containing one or more N-H bonds, as well as to methods of making and using such prodrugs. The particular embodiments described herein are intended in all respects to be illustrative rather than restrictive. Alternative embodiments will become apparent to those skilled in the art to which the present invention pertains without departing from its scope.

The present invention is drawn to sulfenamide prodrugs of pharmaceutical compounds containing one or more N-H bonds formed through a reversible derivatization to yield prodrugs with potentially optimized stability, solubility, cell membrane permeability, pharmacokinetic properties and other pharmaceutical properties, depending upon the choice of promoiety. As used herein, the term "sulfenamide" refers to the broad class of N-S bond-containing compounds, wherein the S is bivalent. Similarly, "sulfenamide prodrug(s)" refers to prodrug(s) having a N-S bond, wherein the S is bivalent. This unique utilization of N-S bond chemistry is applicable to the production of derivatives of various pharmaceutical compounds containing one or more N-H bonds and provides a general method for enhancing the chemical stability, solubility, cell membrane permeability, pharmacokinetic properties and other pharmaceutical properties of these classes of compounds to produce the pharmaceutical activity upon in vivo conversion to the parent molecule, thus facilitating their clinical use. This unique utilization of N-S bond chemistry also may provide a means for enhancing drug targeting as the reversion of these N-S bond-containing prodrugs can involve the reaction of the prodrug with free sulfhydryl containing compounds (e.g., glutathione or biological cysteine) which are present in high concentration in certain tissues or at particular sites. As such, the pharmaceutical compound may be delivered selectively to those tissues/sites rich in glutathione or other thiol-containing molecules.

The following depicts a general scheme of the reversion of a prodrug to the parent pharmaceutical compound by showing the reaction of the prodrug with thiol containing molecules. This reaction is rapid and quantitative. R¹ is a substituent of an endogenous thiol-containing molecule. R is a substituent of a sulfur-containing promoeity and is described below. R₁ and R₂ are substituents of the parent compound and also are described below.

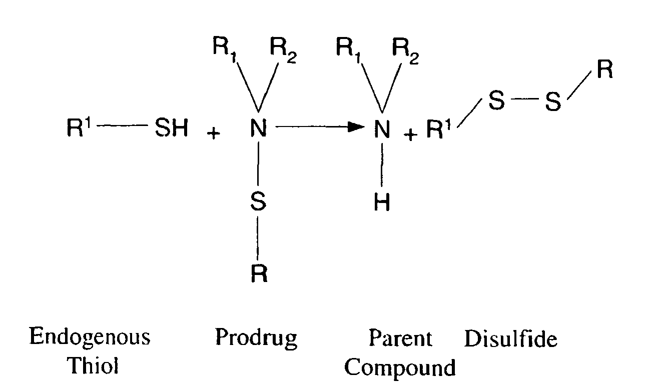

The following depicts an example the possible reversion of a prodrug to a linezolid pharmaceutical compound by showing the reaction of the prodrug with thiol containing molecules. This reaction is rapid and quantitative

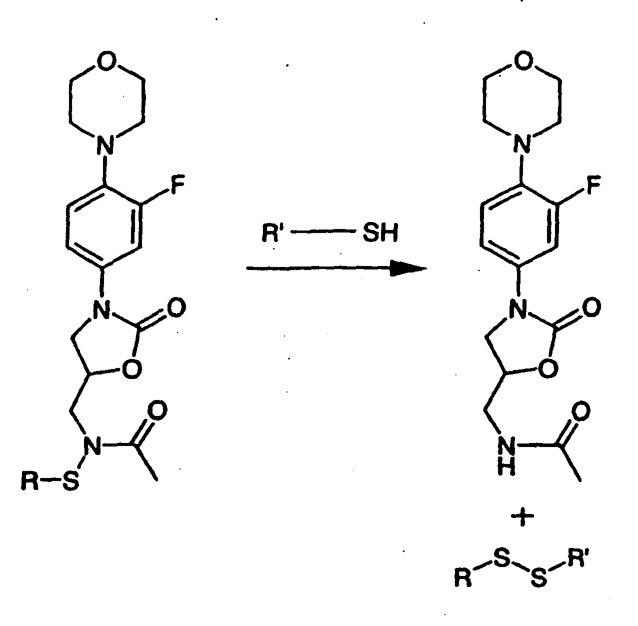

Formula I, above, depicts the general structure of sulfenamide prodrugs of the present invention wherein R₁ and R₂ are substituents of the parent compound. The remainder of the structure represents the sulfur-containing promoiety.

Pharmaceutical compound, as used herein, includes, but is not limited to, substance(s) for the treatment and diagnosis of disease states of humans and animals. Pharmaceutical compounds containing one or more N-H bonds which are contemplated as being useful in the present invention and which maybe converted to sulfenamide prodrugs using one or more of the above-illustrated Reaction Schemes include the following:
C) Uracils.. Parent uracil compounds have a representative structure as shown in Formula XI.

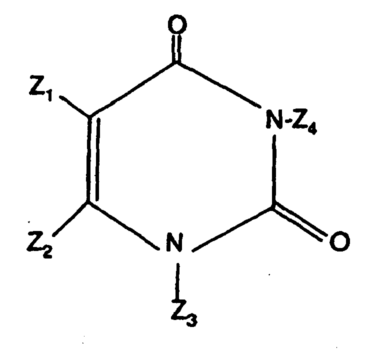

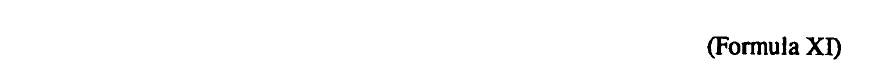

In Formula XI, Z₁- Z₄ are substituents of the parent uracil compound. Each of R and Z₁-Z₄ may be the same or different and each may be hydrogen or any organic or inorganic residue. R is a substituent of a sulfur-containing promoeity, as described above. However, one of Z₃ or Z₄, or both Z₃ and Z₄ must be hydrogen.

Z₁-Z₄, as substituents of the parent uracil compound, may include, but are not limited to,
1. straight-chain substituted or unsubstituted aliphatic groups, such as -CH₃, CH₂CH₃, etc., with or without any additional polar or non-polar functional groups and/or heteroatoms;
2. branched substituted or unsubstituted aliphatic groups, with or without any additional polar or non-polar functional groups and/or heteroatoms;
3. substituted or unsubstituted acyl groups, with or without any additional polar or non-polar functional groups and/or heteroatoms;
5. substituted or unsubstituted aliphatic cyclic groups, such as cyclohexane, etc., with or without any additional polar or non-polar functional groups and/or heteroatoms; and
6. any combination of 1), 2), 3), and 5) with or without additional polar or non-polar functional groups and/or heteroatoms.

Compounds of the general structure of Formula XI, react to form sulfenamide prodrugs having a general structure as illustrated in Formula XII, 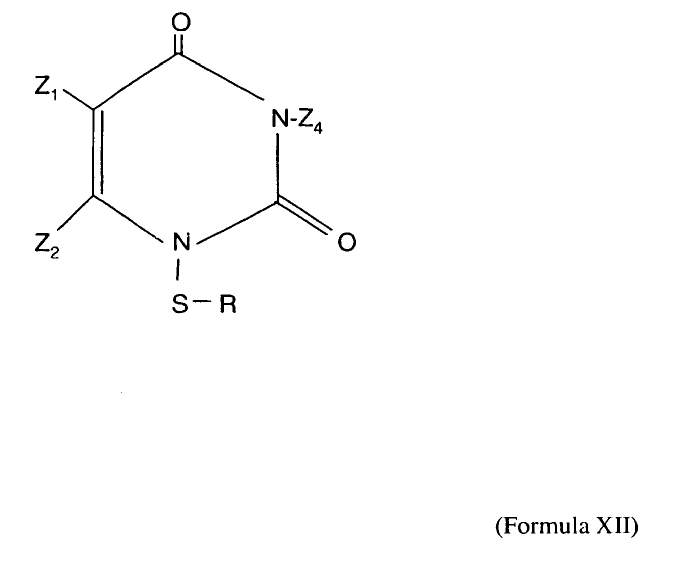 or Formula XIII.

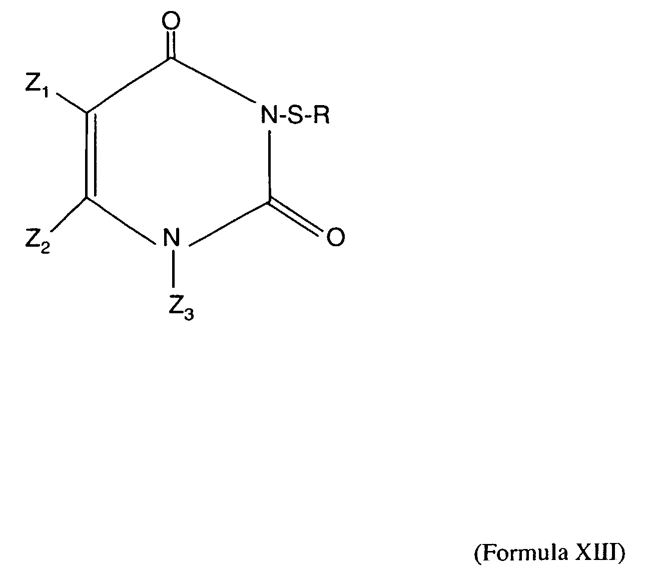

To facilitate reaction of either Z₃ to form a sulfenamide prodrug of Formula XII, or Z₄ to form a sulfenamide prodrug of Formula XIII, the alternative reaction site may be protected by any means known to those of skill in the art.

Examples of pharmaceutical compounds which may be classified as uracils include, but are not limited to, lumazine, , lumichrome, 5-fluorouracil and ketanserin.
D) Barbitals. Parent barbital compounds have a representative structure as shown in Formula XIV.

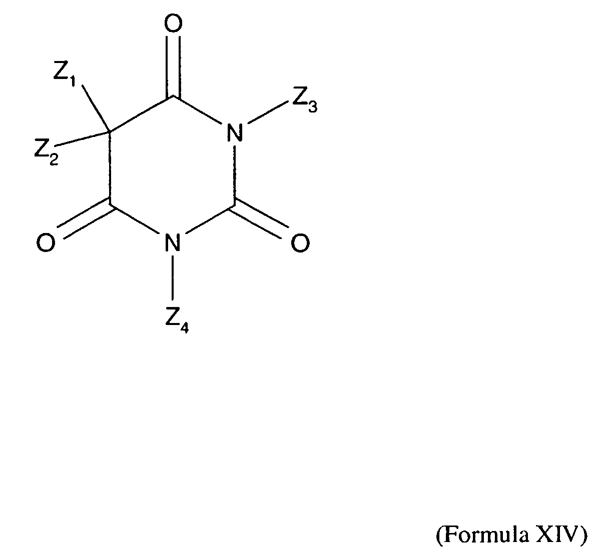

In Formula XIV, Z₁- Z₄ are substituents of the parent barbital compound. Each of R and Z₁-Z₄ may be the same or different and each may be hydrogen or any organic or inorganic residue. R is a substitutent of a sulfur-containing promoeity as described above. One of Z₃ or Z₄, or both Z₃ and Z₄ must be hydrogen.

Z₁-Z₄, as substituents of the parent barbital compound, may include, but are not limited to:
1. straight-chain substituted or unsubstituted aliphatic groups, such as -CH₃, CH₂CH₃, etc., with or without any additional polar or non-polar functional groups and/or heteroatoms;
2. branched substituted or unsubstituted aliphatic groups, with or without any additional polar or non-polar functional groups and/or heteroatoms;
3. substituted or unsubstituted acyl groups, with or without any additional polar or non-polar functional groups and/or heteroatoms;
5. substituted or unsubstituted aliphatic cyclic groups, such as cyclohexane, etc., with or without any additional polar or non-polar functional groups and/or heteroatoms; and
6. any combination of 1), 2), 3), and 5) with or without additional polar or non-polar functional groups and/or heteroatoms.

Compounds of the general structure of Formula XIV, react to form sulfenamide prodrugs having a general structure as illustrated in Formula XV, 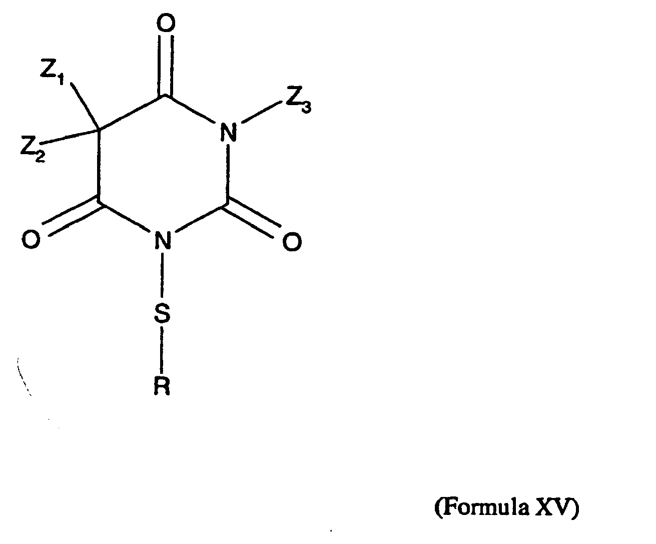 or Formula XVI.

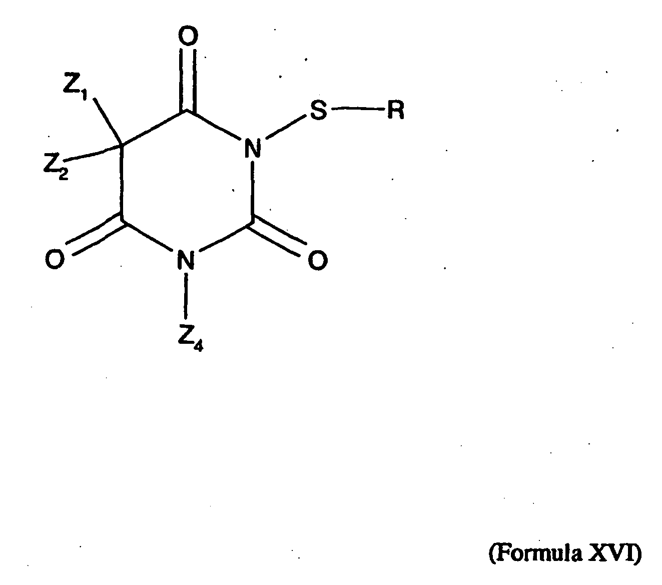

To facilitate reaction of either Z₅ to form a sulfenamide prodrug of Formula XV, or Z₆ to form a sulfenamide prodrug of Formula XVI, the alternative reaction site may be protected by any means known to those of skill in the art.

Examples of pharmaceutical compounds which may be classified as barbitals include, but are not limited to, mephobarbital and heptabarbital, febarbamate and carbubarb.
E) Peptides. Parent amide compounds having the representative structure illustrated in Formula XVII, 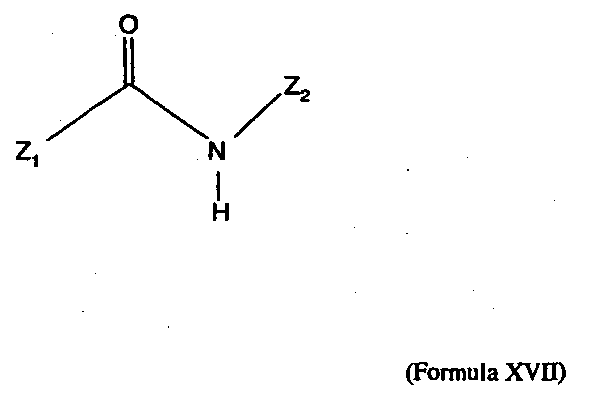 react to form sulfenamide prodrugs having a general structure as illustrated in Formula XVIII.

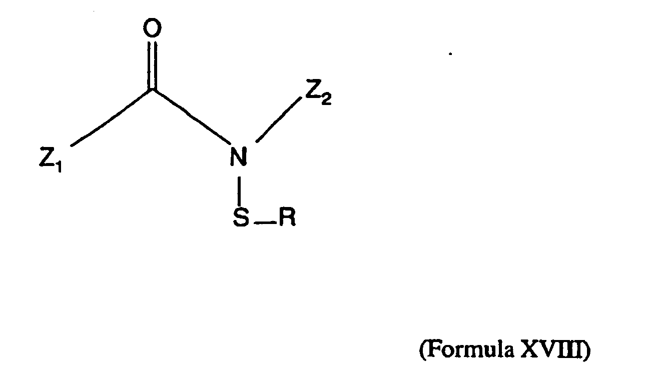

In each of Formulas XVII and XVIII, Z₁ and Z₂ are substituents of the parent amide or peptide compound. R, Z₁ and Z₂ may be the same or different and each may be hydrogen or any organic or inorganic residue. R is a substituent of the sulfur-containing promoiety, as described above.

Z₁ and Z₂, as substituents of the parent amide or peptide compound, may include, but are not limited to:
1. straight-chain substituted or unsubstituted aliphatic groups, such as -CH₃, CH₂CH₃, etc., with or without any additional polar or non-polar functional groups and/or heteroatoms;
2. branched substituted or unsubstituted aliphatic groups, with or without any additional polar or non-polar functional groups and/or heteroatoms;
3. Z₂ may be a substituted or unsubstituted acyl group, with or without any additional polar or non-polar functional groups and/or heteroatoms;
5. substituted or unsubstituted aliphatic cyclic groups, such as cyclohexane, etc., with or without any additional polar or non-polar functional groups and/or heteroatoms; and
6. any combination of 1), 2), 3), and 5) with or without additional polar or non-polar functional groups and/or heteroatoms.

Examples of pharmaceutical compounds with this classification include, but are not limited to, hydroorotic acid, lumazine, diflubenzuron, biuret, apronalide, carbomal, lumichrome, phenytoin, allantoin, 5-fluorouracil, ethotoin, ketanserin, carbamazepine, nevirapine, indinavir, benzamide (model compound), acetanilide, linezolid, paclitaxel, thalidomide and any peptide drug with an available amide N-H bond.
F) Ureas: Parent urea compounds have a representative structure as shown in Formula XIX.

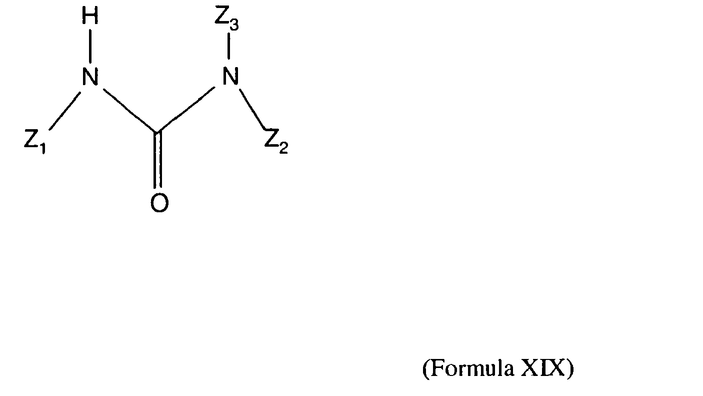

Compounds of the general structure of Formula XIX, react to form sulfenamide prodrugs having a general structure as illustrated in Formula XX.

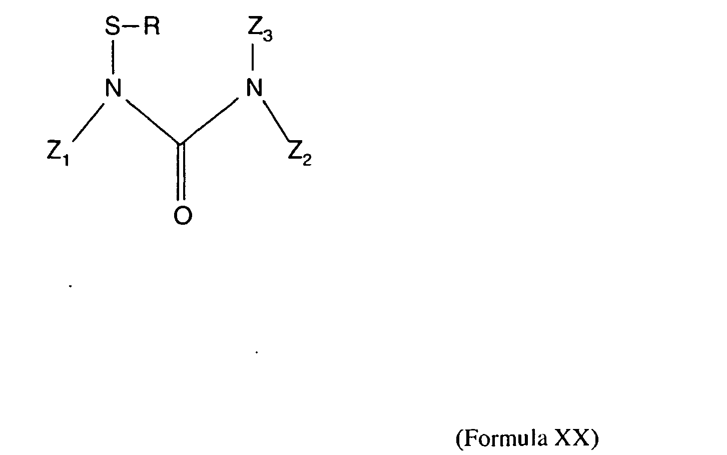

In each of Formulas XIX and XX, Z₁ - Z₃ are substituents of the parent urea compound. Each of R, Z₁ - Z₃ may be the same or different and each may be hydrogen or any organic or inorganic residue. R is a substituent of the sulfur-containing promoiety as described above. If either Z₂ or Z₃, or both Z₂ and Z₃ are hydrogen resulting in a compound having multiple potential reaction sites, the sites at which reaction is not desired may be protected by any means known to those of skill in the art.

Z₁-Z₃, as substituents of the parent urea compound, may include, but are not limited to:
1. straight-chain substituted or unsubstituted aliphatic groups, such as -CH₃, CH₂CH₃, etc., with or without any additional polar or non-polar functional groups and/or heteroatoms;
2. branched substituted or unsubstituted aliphatic groups, with or without any additional polar or non-polar functional groups and/or heteroatoms;

Examples of pharmaceutical compounds with this classification, include but are not limited to: hydroorotic acid, lumazine, diflubenzuron, biuret, apronalide, carbromal, lumichrome, phenytoin, allantoin, 5-fluorouracil, ethotoin, ketanserin and carbamazepine.

Suitable pharmaceutical carriers for the embodiment of the present invention include any aqueous or non-aqueous carriers which are useful for administration of the drug or prodrug, preferably those which are non-toxic, otherwise inert, medically acceptable and compatible with the prodrug. Particularly useful are buffer saline based carriers. The present compositions may further comprise other active ingredients such as antimicrobial agents and other agents such as preservatives, solubilizers and stabilizers.

The present prodrug may also be in a solid form. The solid form may be a tablet, dry powder or granules. The solid form may also contain a suitable binder material, coating agent or other ingredients that facilitate formulation.

The present compositions may be administered though a variety of routes of administration. These routes include, but are not limited to, parenteral, oral, intramuscular, subcutaneous, nasal, dermal, ophthalmic, inhalation, pulmonary, vaginal, rectal and aural routes.

Some of the following are examples of methods of making N-S bond-containing compounds according to the present invention. It will be understood and appreciated that the following examples are merely exemplary and not intended to limit the invention in any way.

### Example 1

### Synthesis of Phthalimide-Cysteine Derivatives

Phthalimide is an N-H bond-containing imide compound and the model compound utilized for development of the present invention. Cysteine is a sulfur-containing molecule that has been determined to react with a variety of N-H bond-containing compounds, including Phthalimide. Various derivatives of cysteine have also been found to be suitable derivatizing agents for the N-S chemistry of the present invention.

The following reaction was performed entirely under anhydrous conditions and in an inert atmosphere (argon, etc.). First, 1.1 equivalents of an appropriate halogenating agent (SO₂Cl₂, Br₂, etc.) were dissolved in an appropriate amount of a suitable solvent such as 1,2 Dichloroethane (DCE). Subsequently, the solution was cooled to a temperature in the range of -15°C to 0°C. The solution was then transferred dropwise to a solution or suspension containing 1 equivalent of a derivative of cystine and a suitable solvent such as DCE, where both the carboxylic acid groups and amino groups have been protected with suitable protecting groups (e.g., TFA, Cbz, Boc, Methyl ester, Ethyl ester, t-Butyl ester, etc.). The solution or suspension was also at a temperature of -15°C to 0°C. The resulting mixture was then agitated for between 5 and 30 minutes and subsequently transferred dropwise to a suspension of potassium phthalimide (2 equivalents) in a suitable solvent such as DCE at - 15°C to 0°C. This resulting mixture was agitated at -15°C to 0°C for between 10 minutes and one hour and subsequently allowed to slowly warm to ambient temperature. The reaction was stopped when no further change appeared in the TLC analysis. The precipitate is removed either by filtration or centrifugation and the filtrate or supernatant was concentrated to yield the crude residue. The desired product was isolated by column chromatography and/or recrystallization. Any necessary deprotection was subsequently conducted and purification (if necessary) of the deprotected product was performed by column chromatography and/or recrystallization.

### Example 2

### Synthesis of Aniline-Cysteine Derivatives

The reaction was performed entirely under anhydrous conditions and in an inert atmosphere (argon, etc.). First, 1.1 equivalents of an appropriate halogenating agent (SO₂Cl₂, Br₂, etc.) was dissolved in an appropriate amount of a suitable solvent such as 1,2 Dichloroethane (DCE). Subsequently, the solution was cooled to a temperature in the range of -15°C to 0°C. The solution then was transferred dropwise to a solution or suspension containing one equivalent of a cystine derivative and a suitable solvent such as DCE, where both the carboxylic acid groups and amino groups have been protected with suitable protecting groups (TFA, Cbz, Boc, Methyl ester, Ethyl ester, t-Butyl ester, etc.). The solution or suspension also was at a temperature of -15°C to 0°C. The resulting mixture was agitated for between 5 and 30 minutes and then was transferred dropwise to a solution containing four equivalents of aniline and a suitable solvent such as DCE at -15°C to 0°C. The resulting mixture was agitated at - 15°C to 0°C for between 10 minutes and one hour, and subsequently allowed to slowly warm to ambient temperature. The reaction was stopped when no further change appeared in the TLC analysis. The precipitate was removed either by filtration or centrifugation and the filtrate or supernatant was concentrated to give the crude residue. The desired product subsequently was isolated by column chromatography and/or recrystallization.

### Example 3

### Synthesis of Phthalimide-Dithiocarbamate

The reaction was performed entirely under anhydrous conditions and in an inert atmosphere (argon, etc.). A solution of N-Bromo Phthalimide (1 equivalent) in DMF at 0°C was added dropwise to a solution of Sodium Dithiocarbamate (1 equivalent) in DMF at 0°C. The mixture was agitated for one hour at 0°C and then for one hour at 10-15°C. The reaction mixture was concentrated, the crude taken up into acetone and the precipitate filtered. The desired product was isolated by a combination of column chromatography and recrystallization.

### Example 4

### Synthesis of Benzamide-Cysteine Derivatives

Initially, the sodium salt of benzamide was made in situ by reacting benzamide with sodium hydride in a suitable solvent such as tetrohydrofuran (THF). The reaction was allowed to proceed for 45 minutes at 0°C followed by 30 minutes at ambient temperature. The sodium salt of benzamide was subsequently set aside under argon until needed in the reaction.

The reaction was performed entirely under anhydrous conditions and in an inert atmosphere (argon, etc.). First, 1.1 equivalents of an appropriate halogenating agent (e.g., SO₂Cl₂, Br₂, etc.) was dissolved in an appropriate amount of a suitable solvent such as 1,2 Dichloroethane (DCE). Subsequently, the solution was cooled to a temperature in the range of -15°C to 0°C. The solution was then transferred dropwise to a solution or suspension containing one equivalent of the chosen derivative of cystine and a suitable solvent such as DCE, where both the carboxylic acid groups and amino groups have been protected with suitable protecting groups (e.g., TFA, Cbz, Boc, Methyl ester, Ethyl ester, t-Butyl ester, etc.). The solution or suspension also was at a temperature of -15°C to 0°C. The resulting mixture was agitated for between 5 and 30 minutes and then was transferred dropwise to a suspension containing two equivalents of the sodium salt of benzamide and a suitable solvent such as DCE at -15°C to 0°C. The resulting mixture was agitated at -15°C to 0°C for between 10 minutes and one hour, and subsequently allowed to slowly warm to ambient temperature. The reaction was stopped when no further change appeared in the TLC analysis. The precipitate was removed either by filtration or centrifugation and the filtrate or supernatant was concentrated to give the crude residue. The desired product subsequently was isolated by column chromatography and/or recrystallization. Any necessary deprotection then was conducted and purification (if necessary) of the deprotected product was performed by column chromatography and/or recrystallization.

### Example 5

### Synthesis of Flutamide-Cysteine Derivatives

Initially, the sodium salt of flutamide was made in situ by reacting flutamide with sodium hydride in a suitable solvent such as tetrohydrofuran (THF). The reaction was allowed to proceed for 45 minutes at 0°C followed by 30 minutes at ambient temperature. The sodium salt of flutamide was subsequently set aside under argon conditions until needed in the reaction.

The reaction was performed entirely under anhydrous conditions and in an inert atmosphere (argon, etc.). First, 1.1 equivalents of an appropriate halogenating agent (e.g., SO₂Cl₂, Br₂, etc.) was dissolved in an appropriate amount of a suitable solvent such as 1,2 Dichloroethane (DCE). Subsequently, the solution was cooled to a temperature in the range of -15°C to 0°C. The solution was then transferred dropwise to a solution or suspension containing one equivalent of the chosen derivative of cysteine and a suitable solvent such as DCE, where both the carboxylic acid groups and amino groups have been protected with suitable protecting groups (e.g., TFA, Cbz, Boc, Methyl ester, Ethyl ester, t-Butyl ester, etc.). The solution or suspension also was at a temperature of -15°C to 0°C. The resulting mixture was agitated for between 5 and 30 minutes and then was transferred dropwise to a suspension containing two equivalents of the sodium salt of flutamide and a suitable solvent such as DCE at -15°C to 0°C. The resulting mixture was agitated at -15°C to 0°C for between 10 minutes and one hour, and subsequently allowed to slowly warm to ambient temperature. The reaction was stopped when no further change appeared in the TLC analysis. The precipitate was removed either by filtration or centrifugation and the filtrate or supernatant was concentrated to give the crude residue. The desired product subsequently was isolated by column chromatography and/or recrystallization. Any necessary deprotection then was conducted and purification (if necessary) of the deprotected product was performed by column chromatography and/or recrystallization.

### Example 6

The following method was used to make N-S Bond-containing prodrugs from amide compounds such as, carbamazepine, acetanilide, flutamide, benzamides, N-methylbenzamide, phenylcarbamate and paclitaxel. This method is also applicable to amides that dissolve in THF.

Sulfuryl chloride (1 mmol) (1M solution in CH2Cl2) was added to pure diaryl/dialiphaticdisulfide (1 mmol) and cooled to 0°C. The resultant solution was stirred at 0°C for 1 hour to yield a deep yellow solution of the aryl/aliphaticsulfenyl chloride. The cold (0°C) aryl/aliphaticsulfenyl chloride generated above was transferred, via syringe, to a cold (0°C) solution of the amide (1 mmol) containing Et₃N (1 mmol) in THF (5mL). Immediately after addition, the appearance of a white precipitate was observed. This suspension was stirred at 0°C for 1.5 hours. Water (2 mL) followed by ethyl acetate (10 mL) was added to the suspension and the layers were separated. The separated organic phase was dried over anhydrous Na₂SO₄ and evaporated in a rotavap below 40°C to yield crude material. Flash column chromatography, using hexane/ethyl acetate as the eluent, of the crude material yielded the pure sulfenamide The percentage of each solvent used varied based on the polarity of the product.

### Example 7

The following method was used to make N-S Bond-containing prodrugs from acetanilide. This method is also applicable to amides that dissolve in acetonitrile.

Sulfuryl chloride (1 mmol) (1M solution in CH₂Cl₂) was added to pure diaryl/dialiphaticdisulfide (1 mmol) and cooled to 0°C. The resultant solution was stirred at 0°C for 1 hour to yield a deep yellow solution of the aryl/aliphaticsulfenyl chloride. The cold (0°C) aryl/aliphaticsulfenyl chloride generated above was transferred, via syringe, to a cold (0°C) solution of the amide (1 mmol) containing Et₃N (1 mmol) in acetonitrile (5 mL). Immediately after addition, the appearance of a white precipitate was observed. This suspension was stirred at 0°C for 1.5 hours. Water (2 mL) followed by ethyl acetate (10 mL) was added to the suspension and the layers were separated. The separated organic phase was dried over anhydrous Na₂SO₄ and evaporated in a rotavap below 40°C to yield crude material. Flash column chromatography, using hexane/ethyl acetate as the eluent, of the crude material yielded the pure sulfenamide. The percentage of each solvent used varied based on the polarity of the product.

### Example 8

The following method was used to make N-S Bond-containing prodrugs from linezolid. This method also is applicable to amides that do not readily dissolve in THF.

Sulfuryl chloride (1 mmol) (1M solution in CH₂Cl₂) was added to pure diaryl/dialiphaticdisulfide (1 mmol) and cooled to 0°C. The resultant solution was stirred at 0°C for 1 hour to yield a deep yellow solution of the aryl/aliphaticsulfenyl chloride. The cold (0°C) aryl/aliphaticsulfenyl chloride generated above was transferred, via syringe, to a cold (0°C) solution of the amide (1 mmol) containing Et₃N (1 mmol) in a solvent mixture of dimethylformamide (DMF) (1 mL) tetrahydrofuran (THF) (10 mL). Immediately after addition, the appearance of a white precipitate was observed. This suspension was stirred at 0°C for 1.5 hours. Water (2 mL) followed by ethyl acetate (10 mL) was added and the layers were separated. The separated organic phase was dried over anhydrous Na₂SO₄ and evaporated in a rotavap below 40°C to yield a crude material. Flash column chromatography, using hexane/ethyl acetate was used as eluent, of the crude material yielded the pure sulfenamide. The percentage of each solvent used varied based on the polarity of the product.

### Example 9

The following compound was synthesized using the method described in Example 6 and alternatively using the example described in Example 7. The compound is a prodrug of the parent N-H bond-containing pharmaceutical compound acetanilide.

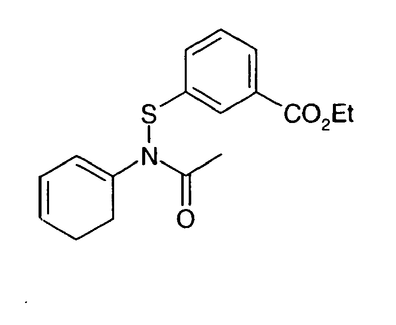

Separation by column chromatography on silica gel using hexane/ethyl acetate (4:1) as eluent gave a white solid (55%). ¹H NMR (400 MHz, CDCl₃): δ 1.38 (t, 3H.*J* = 8 Hz) 2. 30 (s, 3H), 4.39 (q,2H,*J* = 8 Hz), 7.20-7.53 (series of m, 7H), 7.90 (m, 2H); ¹³C (100 MHz, CDCl₃): δ 61.3, 137.1, 144.9, 164.9; HRMS: calculated for [MH]⁺ C₁₇H₁₈NO₃S 316.1007; found 316.0993.

### Example 10

A prodrug from parent N-H bond-containing pharmaceutical compound linezolid was synthesized using the method described in Example 8 above. Linezolid has the following properties: white crystalline solid, m.p. = 180.1 °C; R_{f} = 0 (eluent: 60% ethyl acetate/hexane) - polar; solubility in soybean oil ∼ 0.5 mg/ml- poorly soluble. The following is the structure of the parent N-H bond-containing pharmaceutical compound linezolid:

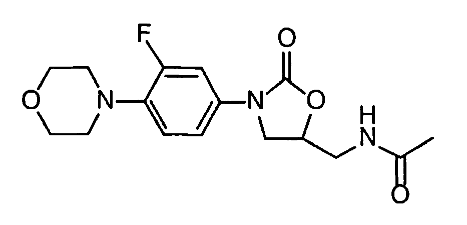

The following is the structure of the prodrug synthesized from the parent N-H bond-containing pharmaceutical compound linezolid.

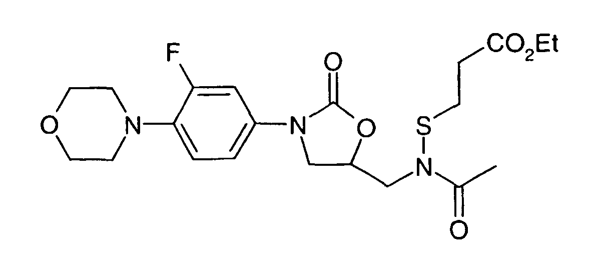

Separation by column chromatography on silica gel using hexane/ethyl acetate as eluent (3:2) gave a colorless viscous oil (72%). ¹H NMR (400 MHz, CDCl₃): δ 1.22 (t, 3H. J = 8 Hz,), 2.39 (s, 3H), 2.65 (m, 2H), 2.98-3.25 (broad m, 4H), 3.68 (m, 2H), 3.88 (m, 4H), 3.99-4.19 (broad m, 6H), 4.86 (m, 1H), 6.90 (d d, 1H, J = 2Hz), 7.08 (d, 1H, J = 2 Hz), 7.40 (d, 1H, J = 6 Hz); ¹³C (100 MHz,CDCl₃):δ 23.1, 30.4, 38.1, 48.3, 51.5, 57.1, 60.0, 61.4, 67.3, 70.9, 107.5, 108.5, 11 4.1, 117.8, 133.2, 137.1, 154.7, 154.9, 156.8, 170.2, 177.1; HRMS: calculated for [MH]⁺ C₂₁H₂₈O₆N₃SF 470.1765; found 470.1735. The prodrug of this example had the following optimized properties: viscous oil; R_{f} = 0.25 (eluent (60% ethyl acetate/hexane) - non-polar; solubility in soybean oil > 30 mg/ml.

### Example 11

A prodrug from parent N-H bond-containing pharmaceutical compound linezolid was synthesized using the method described in Example 8 above. The parent N-H bond-containing pharmaceutical compound linezolid had the following properties: white crystalline solid; m.p. = 180.1 °C; R_{f} = 0 (eluent: 60% ethyl acetate/hexane)- polar; and solubility in soybean oil - 0.5 mg/ml-poorly soluble; and the following structure

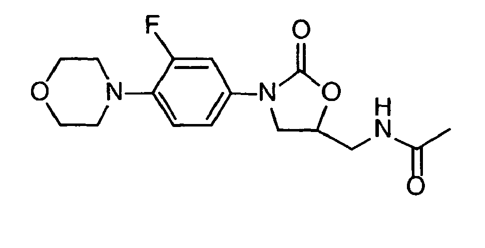

The prodrug synthesized from the parent N-H bond-containing pharmaceutical compound linezolid had the following structure:

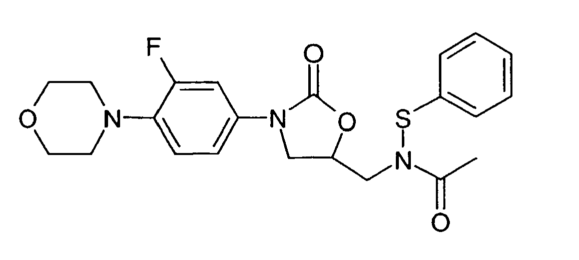

Separation by column chromatography on silica gel using hexane/ethyl acetate (3:2) as eluent (53%) gave a white crystalline solid. ¹H NMR (400 MHz, CDCl₃): δ 2.40 (m, 7H), 2.90 (m, 2H), 3.30 (m, 2H), 3.68 (m, 2H), 3.75-4.12 (m, 2H), 4.88 (m,1H), 7.02 (m, 1H), 7.10-7.30 (m, 4H), 7.41 (m,3H); HRMS: calculated for [MH]₊ C₂₂H₂₅N₃O₄SF 466.1550; found 466.1545. The prodrug of this example had the following optimized properties: white crystalline solid; m.p. = 120.9 °C; R_{f} = 0.4 (eluent 60% ethyl acetate/hexane) -non-polar; solubility in soybean oil > 10 mg/ml.

### Example 12

The following compound was synthesized using the method described in Example 6 and is a prodrug of the parent N-H bond-containing pharmaceutical compound carbamazepine.

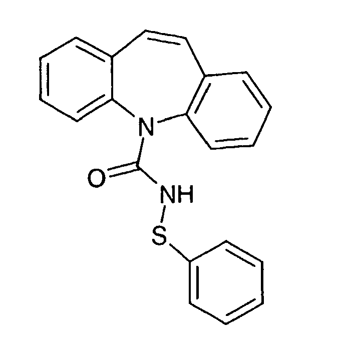

Separation by column chromatography on silica gel using hexane/ethyl acetate (4:1) as eluent (35%) provided a white solid. ¹H NMR (400 MHz, CDCl₃): δ 7.15-7.55 (series of m, 15 H); ¹³C (100 MHz, CDCl₃): δ 134.5, 139.0, 139.8, 156.2; HRMS: calculated for [MH]+ 345.1062; found 345.1063.

### Example 13

The following compound was synthesized using the method described in Example 4 and is a prodrug of the parent N-H bond-containing pharmaceutical model compound benzamide.

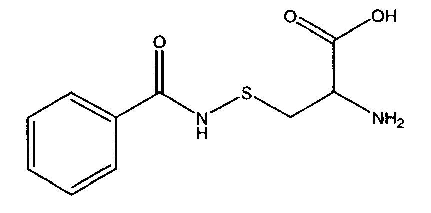

¹HNMR (300MHz, DMSO-d6): δ 3.05 (d of d, 1H), 3.25 (d of d, 1H), 3.80 (broad), 4.15 (broad, 1H), 7.45 (t, 2H), 7.55 (t, 1H), 7.80 (d, 2H), 8.40 (broad), 9.60 (s, 1H). FAB+ 241.0 (M+1). Aqueous Stability: within a pH range of 4.0 to 7.0, the pH of maximum stability (projected by Eyring Plot) is 6.0 at 25 degrees Celsius with half life of - 6.3 years (∼10µg/mL benzamide-cysteine model prodrug solution in 35mM Phosphate Buffer with Ionic Strength or 0.15M obtained by addition of NaCl). Pseudo-first order degradation kinetics were observed. When a 10 times molar excess of cysteine was added to the above solution, the model prodrug degraded (reverted to yield benzamide) with a half life of - 20 to 30 minutes. In addition, the above benzamide-cysteine model prodrug had improved aqueous solubility compared to benzamide.

### Example 14

The following compound was synthesized using the method described in Example 5 and is a prodrug of the parent N-H bond-containing pharmaceutical compound flutamide.

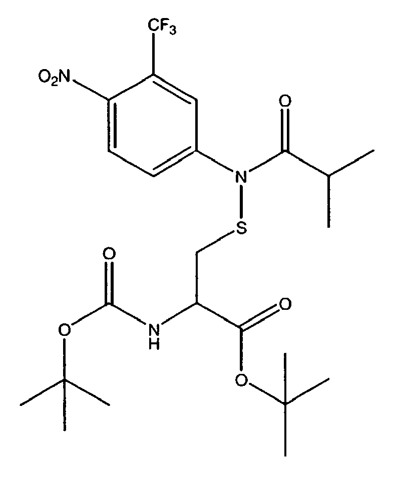

¹HNMR (300MHz, CDCl₃): δ 1.20 (d, 6H), 1.35 (s, 9H), 1.45 (s, 9H), 3.05 (d of d, 1H), 3.25 (d of d, 1H), 3.50 (m, 1H), 4.35 (m, 1H), 5.20 (broad d, 1H), 7.60 (d of d, 1H), 7.75 (s, 1H), 7.90 (d, 1H).

### Example 15

The following compound was synthesized using the method described in Example 1 and is a prodrug of the parent N-H bond-containing pharmaceutical model compound phthalimide.

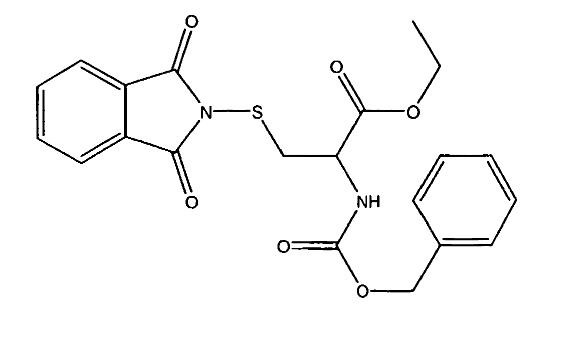

¹HNMR (300MHz, CDCl₃): δ 1.15 (t, 3H), 3.10 (d of d, 1H), 3.70 (d of d, 1H), 3.95 (m, 2H), 4.70 (m, 1H), 5.05 (d of d, 2H), 6.25 (d, 1H), 7.35 (broad s, 5H), 7.75 (m, 2H), 7.90 (m, 2H). Aqueous Stability: within a pH range of 3.5 to 5.0, the pH of maximum stability is 3.5 at 25 degrees Celsius with half life of - 8 to 9 days (∼2µg/mL phthalimide-cysteine model prodrug solution in 35mM Acetate Buffer with Ionic Strength of 0.15M obtained by addition of NaCl). The aqueous degradation kinetics did not show pseudo-first order kinetics. When a 10 times molar excess of cysteine was added to the above solution, the model prodrug degraded (reverted to yield phthalimide) instantaneously (kinetics were not measurable).

### Example 16

The following compound was synthesized using the method described in Example 6 and is a prodrug of the parent N-H bond-containing pharmaceutical compound carbamazepine.

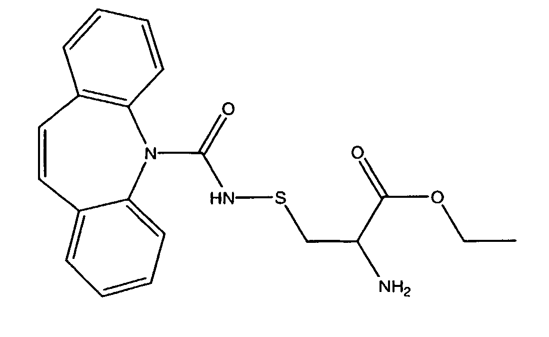

¹HNMR (300MHz, DMSO-d6): δ 1.15 (t, 3H), 2.85 (d of d, 1H), 3.05 (d of d, 1H), 4.05 (m, 1H), 4.15 (q, 2H), 6.95 (s, 2H), 7.10 (s, 1H), 7.35 (m, 2H), 7.40 (broad s, 6H), 8.40 (broad). FAB+ 384.1 (M+1). Aqueous Stability: within a pH range of 4.0 to 7.0, the pH of maximum stability (projected by Eyring Plot) is 4.0 at 25 degrees Celsius with half life of - 181 days (∼2µg/mL carbamazepine prodrug solution in 35mM Acetate Buffer with Ionic Strength of 0.15M obtained by addition of NaCl). Pseudo-first order degradation kinetics were observed. In addition, the above carbamazepine prodrug had greatly improved aqueous solubility compared to carbamazepine.

Once the desired N-S bond-containing compound (i.e, prodrug) has been synthesized, it is placed in a suitable pharmaceutical carrier for delivery into the intended subject. Suitable pharmaceutical carriers for the present invention include any aqueous carriers which are useful for administering the pharmaceutical compounds or prodrugs thereof, preferably those which are non-toxic, otherwise inert, medically acceptable and compatible with the prodrug. Particularly useful are buffer saline based carriers. The present compositions may further comprise other active ingredients such as antimicrobial agents and other agents such as preservatives, solubilizers and stabilizers.

Once successfully delivered to the intended subject, the N-S bond-containing prodrug undergoes a reversion back to the original N-H bond-containing compound from which it was synthesized. Reversion may occur via hydrolysis or via exposure to compounds containing free sulfhydryl groups such as glutathione. Glutathione is present in human whole blood in a concentration of approximately 26.9 to 41.4 mg/100 ml with the mean value approximating 35.4 mg/100 ml. *See* Caren and Caren, Amer. J. Med. Sci., 221, 307 (1951). This concentration is sufficient to create reversion of a large variety of N-S bond-containing prodrugs back to the N-H bond-containing drug from which they were formed.

In conclusion, the present invention is directed to novel prodrugs of various pharmaceutical compounds containing one or more N-H bonds, as well as to methods of making and using such prodrugs. This unique utilization of N-S bond chemistry is applicable to the production of derivatives of various pharmaceutical compounds containing one or more N-H bonds and provides a general method for enhancing the chemical stability, solubility, cell membrane permeability, pharmacokinetic properties and other pharmaceutical properties of these classes of compounds, thus facilitating their clinical use. The present invention has been described in relation to particular embodiments which are intended in all respects to be illustrative rather than restrictive. Alternative embodiments will become apparent to those skilled in the art to which the present invention pertains without departing from its scope.

From the foregoing, it will be seen that this invention is one well adapted to attain all the ends and objects hereinabove set forth together with other advantages which are obvious and which are inherent to the prodrugs and methods of making and using such prodrugs herein disclosed.

Since many possible embodiments may be made of the invention without departing from the scope thereof, it is to be understood that all matter herein set forth or shown in the accompanying drawing is to be interpreted as illustrative and not in a limiting sense.

## Claims

1. A compound having the following formula, 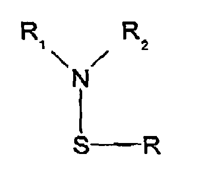 wherein:
R₁ and R₂ are residues of an N-H bond containing pharmaceutical compound being a uracil, a barbital, linezolid, carbamazepine, flutamide, a urea having straight-chain or branched substituted or unsubstituted aliphatic groups with or without any additional polar or non-polar functional groups and/or heteroatoms, or a peptide, and
R is one of a hydrogen, inorganic residue and an organic residue selected from the group consisting of:
a) straight-chain substituted or unsubstituted aliphatic groups, with or without any additional polar or non-polar functional groups and/or heteroatoms;
b) branched substituted or unsubstituted aliphatic groups, with or without any additional polar or non-polar functional groups and/or heteroatoms;
c) substituted or unsubstituted acyl groups, with or without any additional polar or non-polar functional groups and/or heteroatoms;
d) substituted or unsubstituted aliphatic cyclic groups, with or without any additional polar or non-polar functional groups and/or heteroatoms; and
e) any combination of a), b), c), and d) with or without additional polar or non-polar functional groups and/or heteroatoms;
and pharmaceutically acceptable salts thereof.

2. A compound of claim 1, wherein R has the following formula: 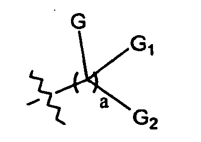
where a is an integer from 0-10 and
wherein a = 0-10; wherein G, G₁, and G₂ may be the same or different and have the following formula: 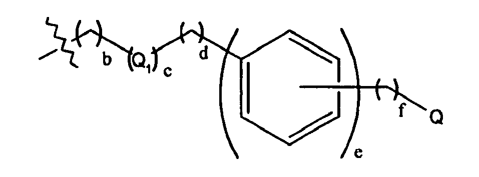
wherein b, c, d, e and f = 0-10; wherein Q₁ is oxygen or sulfur and Q is selected from the group consisting of: 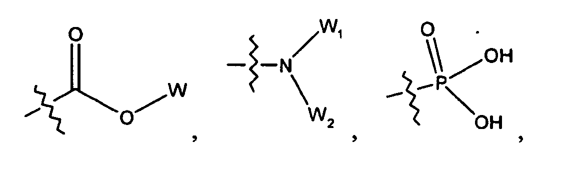 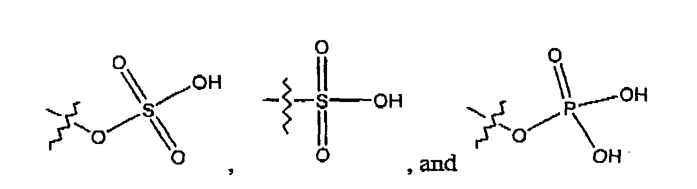
wherein W, W₁ and W₂ each have the same definition as the organic residue of R as defined in claim 1.

3. A compound of claim 1, wherein R is selected from the group consisting of:

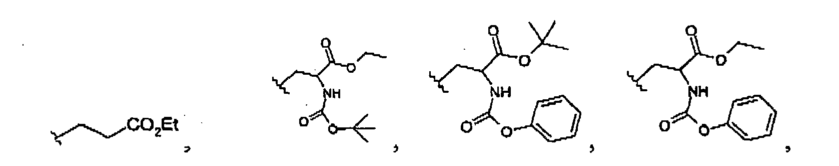

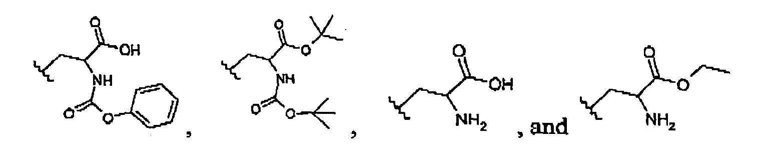

4. A compound of claim 1, wherein R is selected from the group consisting of: 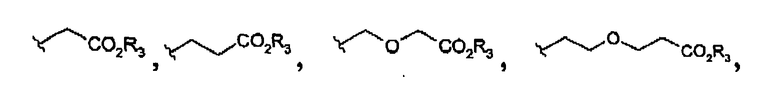 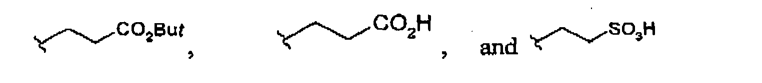
wherein R₃ has the same definition as R as defined in claim 1.

5. A compound of claim 1 being: 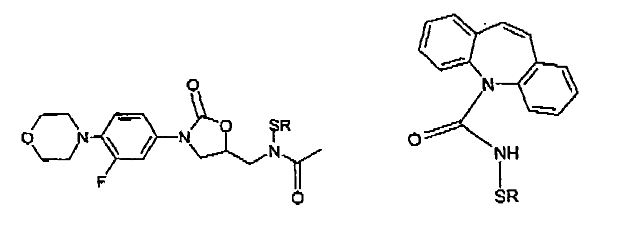 or 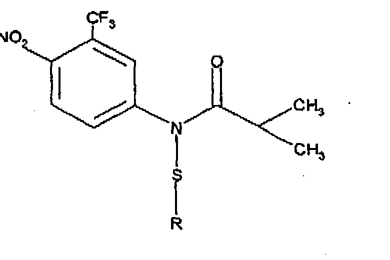 and pharmaceutically acceptable salts thereof.

6. A compound of claim 5 being: 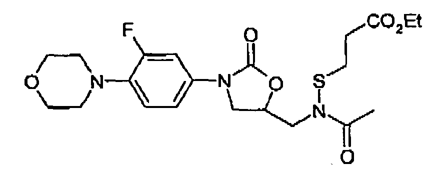 or 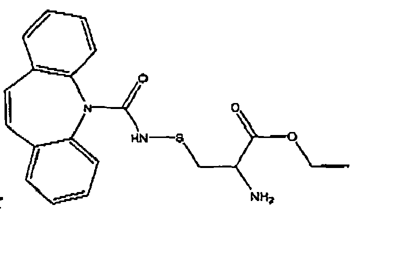 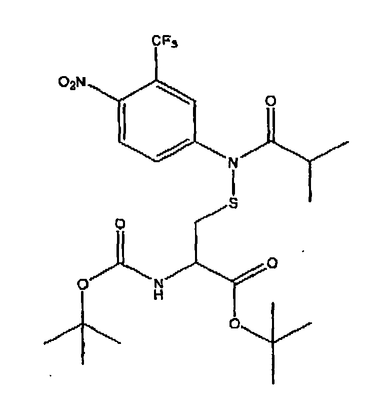 and pharmaceutically acceptable salts thereof.

7. A compound of claim 1 being: 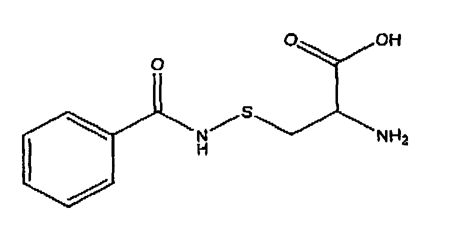 and pharmaceutically acceptable salts thereof.

8. A prodrug being a compound as defined in Claim 1 for use in medicine.

9. A pharmaceutical composition comprising a prodrug as defined in Claim 8 and a pharmaceutically acceptable carrier.

10. A pharmaceutical composition as claimed in claim 9 further comprising an antimicrobial agent, a preservative, a solubilizer, or a stabilizer.

11. A prodrug as claimed in claim 8 being: 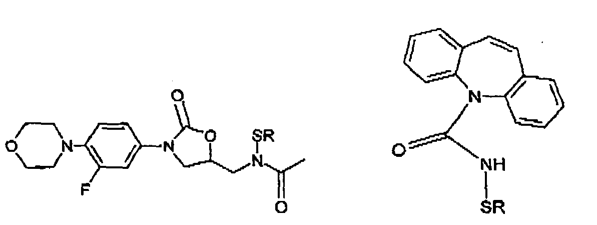 or 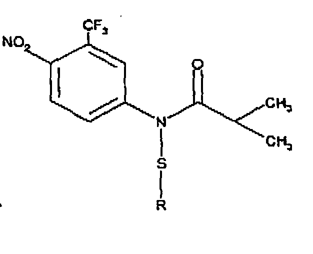 and pharmaceutically acceptable salts thereof.

12. A method of making a prodrug of an N-H bond-containing pharmaceutical compound comprising the reaction illustrated in Reaction Scheme IV 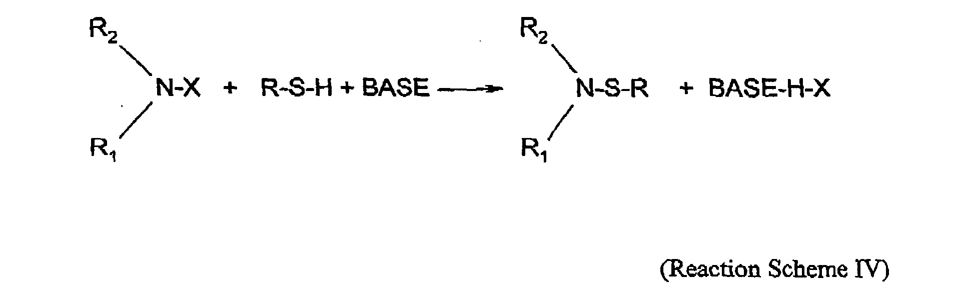
wherein X is any good leaving group,
wherein R₁ and R₂ are residues of an N-H bond-containing pharmaceutical compound, R is as defined in Claim 1.

13. The method of claim 12 wherein the N-H bond-containing pharmaceutical compound is an imide, a hydantoin, a uracil, a barbital, an amide, a benzamide, a N-methylbenzamide, carbamazepine, flutamide, linezolid, a urea, a sulfonamide, an oxazolidinone, or a peptide; or pharmaceutically acceptable salt thereof.

## Patentansprüche

1. Eine Verbindung mit der folgenden Formel 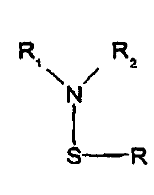 wobei:
R₁ und R₂ Reste einer N-H-Bindung enthaltenden pharmazeutischen Verbindung sind, die ein Uracil, ein Barbital, Linezolid, Carbamazepin, Flutamid, ein Harnstoff mit geradkettigen oder verzweigten substituierten oder nicht substituierten aliphatischen Gruppen mit oder ohne irgendwelche zusätzlichen polaren oder nicht polaren funktionellen Gruppen und/oder Heteroatome oder ein Peptid ist, und
R eines von einem Wasserstoff, einem anorganischen Rest und einem organischen Rest ist, ausgewählt aus der Gruppe, bestehend aus:
a) geradkettigen substituierten oder nicht substituierten aliphatischen Gruppen mit oder ohne irgendwelche zusätzlichen polaren oder nicht polaren funktionellen Gruppen und/oder Heteroatome;
b) verzweigten substituierten oder nicht substituierten aliphatischen Gruppen mit oder ohne irgendwelche zusätzlichen polaren oder nicht polaren funktionellen Gruppen und/oder Heteroatome;
c) substituierten oder nicht substituierten Acyl-Gruppen mit oder ohne irgendwelche zusätzlichen polaren oder nicht polaren funktionellen Gruppen und/oder Heteroatome;
d) substituierten oder nicht substituierten aliphatischen zyklischen Gruppen mit oder ohne irgendwelche zusätzlichen polaren oder nicht polaren funktionellen Gruppen und/oder Heteroatome und
e) einer beliebigen Kombination aus a), b), c) und d) mit oder ohne zusätzliche polare oder nicht polare funktionelle Gruppen und/oder Heteroatome;
und pharmazeutisch akzeptable Salze davon.

2. Verbindung gemäß Anspruch 1, wobei R die folgende Formel aufweist: 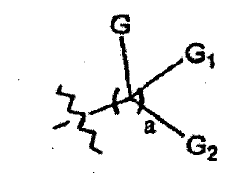
wobei a eine Ganzzahl von 0-10 ist und
wobei a = 0-10, wobei G, G₁ und G₂ gleich oder unterschiedlich sein können und die folgende Formel aufweisen: 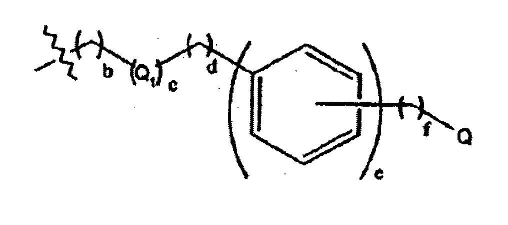
wobei b, c, d, e und f = 0-10; wobei Q₁ Sauerstoff oder Schwefel ist und Q aus der Gruppe ausgewählt ist, bestehend aus: 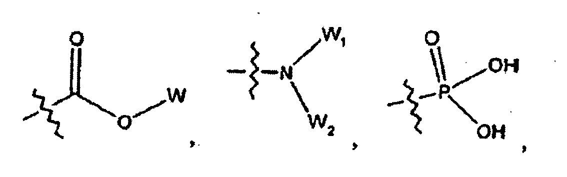 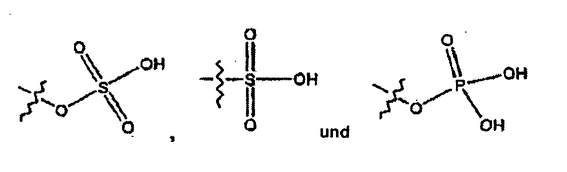
wobei W, W₁ und W₂ jeweils die gleiche Definition wie der in Anspruch 1 definierte organische Rest R aufweisen.

3. Verbindung gemäß Anspruch 1, wobei R aus der Gruppe ausgewählt ist, bestehend aus:

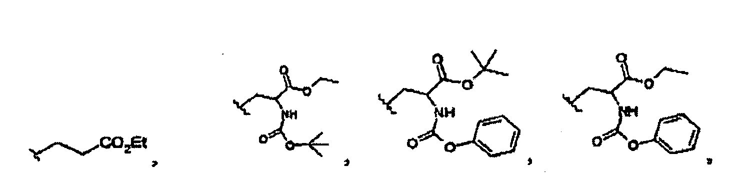

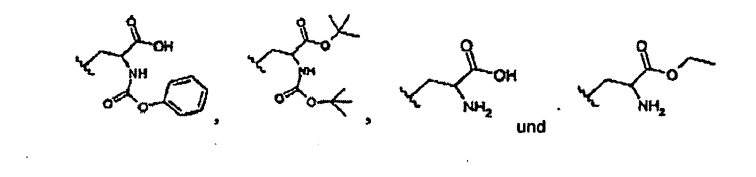

4. Verbindung gemäß Anspruch 1, wobei R aus der Gruppe ausgewählt ist, bestehend aus: 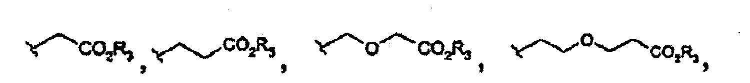 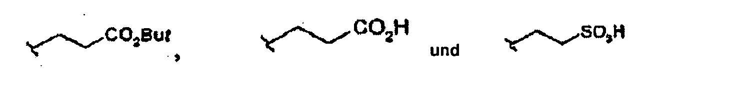
wobei R₃ die gleiche Definition wie R, wie in Anspruch 1 definiert, aufweist.

5. Verbindung gemäß Anspruch 1, die Folgendes ist: 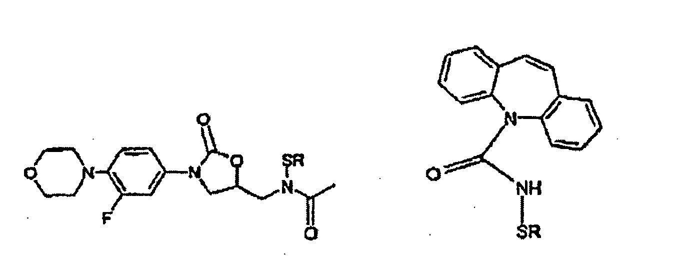 oder 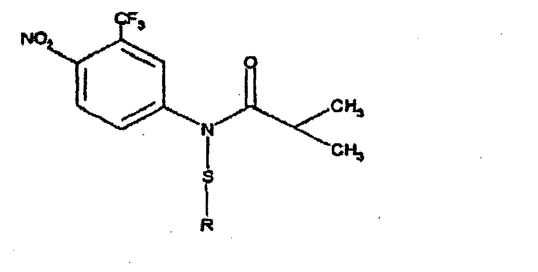 und pharmazeutisch akzeptable Salze davon.

6. Verbindung gemäß Anspruch 5, die Folgendes ist: 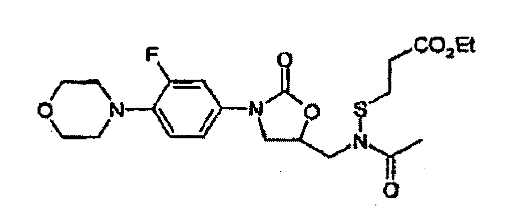 oder 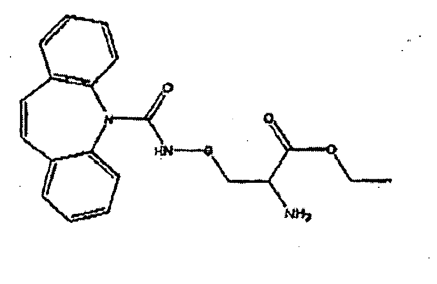 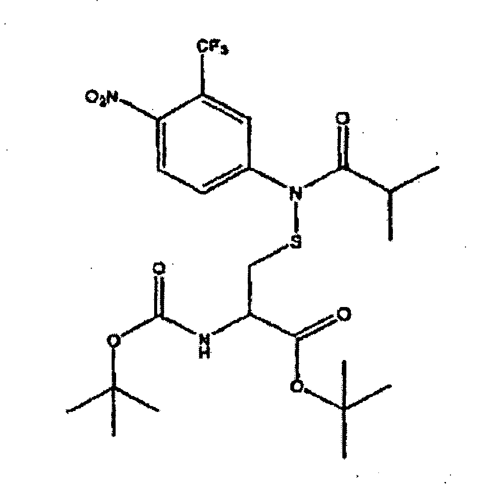 und pharmazeutisch akzeptable Salze davon.

7. Verbindung gemäß Anspruch 1, die Folgendes ist: 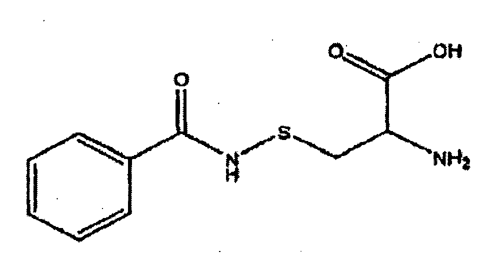 und pharmazeutisch akzeptable Salze davon.

8. Eine Prodrug, die eine wie in Anspruch 1 definierte Verbindung ist, zur Verwendung in der Medizin.

9. Eine pharmazeutische Zusammensetzung, die eine Prodrug, wie in Anspruch 8 definiert, und einen pharmazeutisch akzeptablen Träger beinhaltet.

10. Pharmazeutische Zusammensetzung gemäß Anspruch 9, die ferner einen antimikrobiellen Wirkstoff, einen Konservierungsstoff, einen Lösungsvermittler oder einen Stabilisator beinhaltet.

11. Prodrug gemäß Anspruch 8, die Folgendes ist: 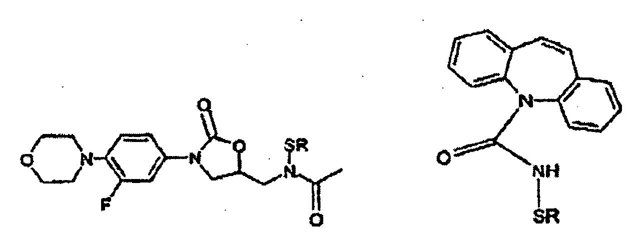 oder 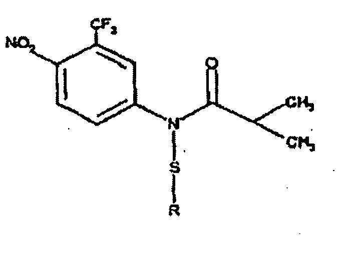 und pharmazeutisch akzeptable Salze davon.

12. Ein Verfahren zum Herstellen einer Prodrug von einer N-H-Bindung enthaltenden pharmazeutischen Verbindung, das die in Reaktionsschema IV dargestellte Reaktion beinhaltet: 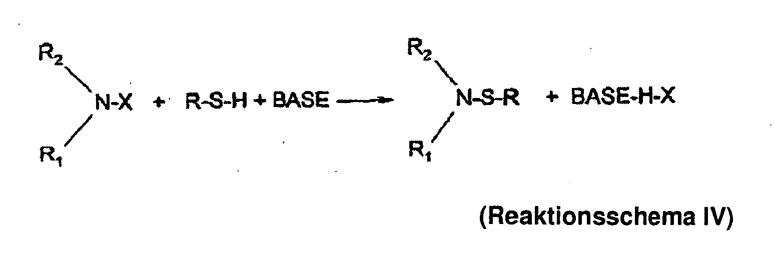
wobei X eine beliebige gute Austrittsgruppe ist,
wobei R₁ und R₂ Reste einer N-H-Bindung enthaltenden pharmazeutischen Verbindung sind, wobei R wie in Anspruch 1 definiert ist.

13. Verfahren gemäß Anspruch 12, wobei die N-H-Bindung enthaltende pharmazeutische Verbindung ein Imid, ein Hydantoin, ein Uracil, ein Barbital, ein Amid, ein Benzamid, ein N-Methylbenzamid, Carbamazepin, Flutamid, Linezolid, ein Harnstoff, ein Sulfonamid, ein Oxazolidinon oder ein Peptid ist; oder ein pharmazeutisch akzeptables Salz davon.

## Revendications

1. Un composé ayant la formule suivante, 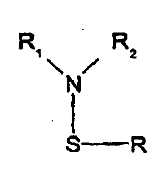 où:
R₁ et R₂ sont des résidus d'un composé pharmaceutique contenant une liaison N-H qui est un uracile, un barbital, du linézolide, carbamazépine, flutamide, une urée ayant des groupes aliphatiques substitués ou non substitués à chaîne droite ou ramifiés avec ou sans groupes fonctionnels polaires ou non polaires et / ou hétéroatomes supplémentaires, ou un peptide, et
R est soit de l'hydrogène, soit un résidu inorganique, soit un résidu organique sélectionné dans le groupe consistant en :
a) groupes aliphatiques substitués ou non substitués à chaîne droite, avec ou sans groupes fonctionnels polaires ou non polaires et / ou hétéroatomes supplémentaires ;
b) groupes aliphatiques substitués ou non substitués ramifiés, avec ou sans groupes fonctionnels polaires ou non polaires et / ou hétéroatomes supplémentaires ;
c) groupes acyles substitués ou non substitués, avec ou sans groupes fonctionnels polaires ou non polaires et / ou hétéroatomes supplémentaires ;
d) groupes cycliques aliphatiques substitués ou non substitués, avec ou sans groupes fonctionnels polaires ou non polaires et / ou hétéroatomes supplémentaires ; et
e) n'importe quelle combinaison de a), b), c) et d) avec ou sans groupes fonctionnels polaires ou non polaires et / ou hétéroatomes supplémentaires ;
et des sels acceptables d'un point de vue pharmaceutique de ceux-ci.

2. Un composé de la revendication 1, dans lequel R a la formule suivante : 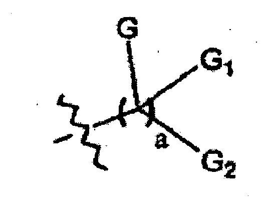
où a est un entier allant de 0 à 10 et
où a = 0 à 10 ; où G, G₁ et G₂ peuvent être semblables ou différents et ont la formule suivante : 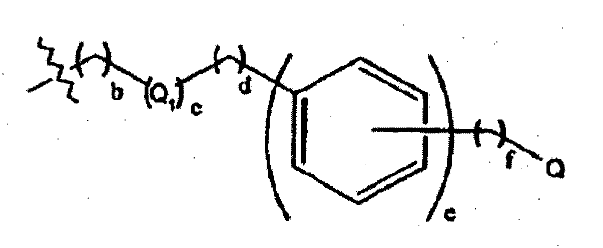
où b, c, d, e et f = 0 à 10 ; où Q₁ est de l'oxygène ou du soufre et Q est sélectionné dans le groupe consistant en : 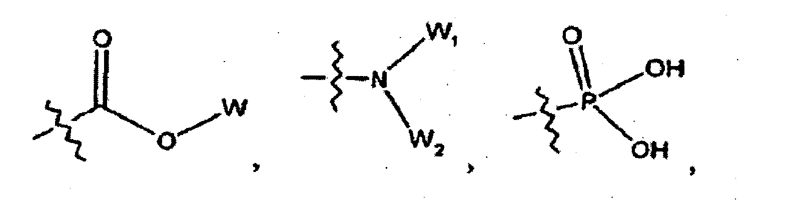 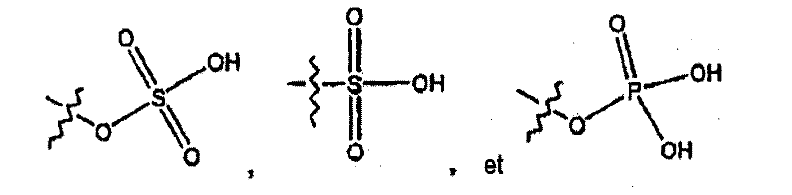
où W, W₁ et W₂ ont chacun la même définition que le résidu organique de R tel que défini dans la revendication 1.

3. Un composé de la revendication 1, dans lequel R est sélectionné dans le groupe consistant en :

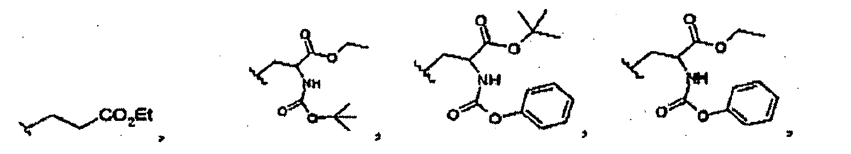

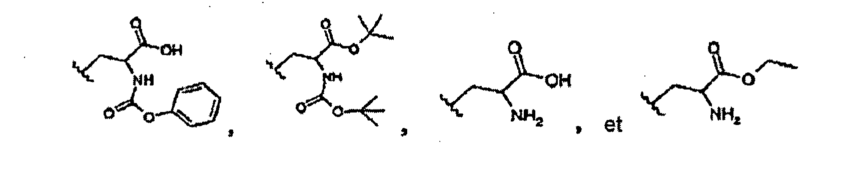

4. Un composé de la revendication 1, dans lequel R est sélectionné dans le groupe consistant en : 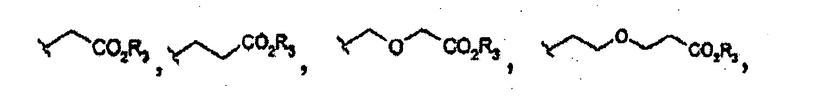 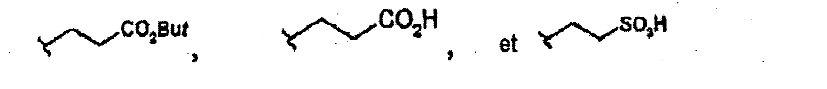
où R₃ a la même définition que R tel que défini dans la revendication 1.

5. Un composé de la revendication 1 qui est : 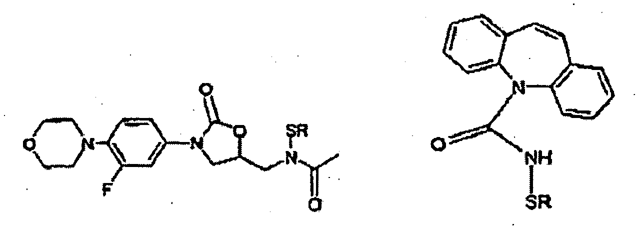 ou 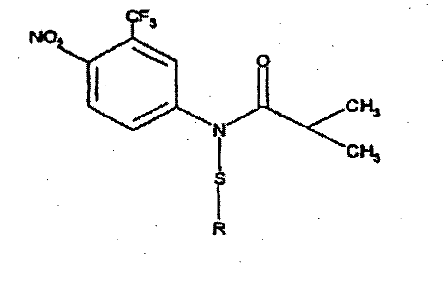 et des sels acceptables d'un point de vue pharmaceutique de celui-ci.

6. Un composé de la revendication 5 qui est : 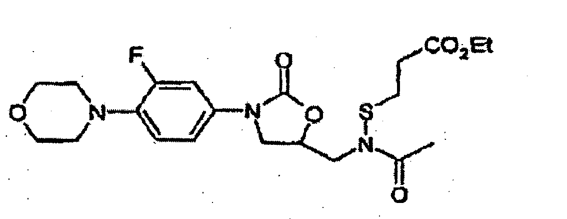 ou 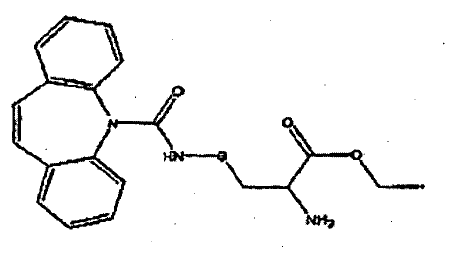 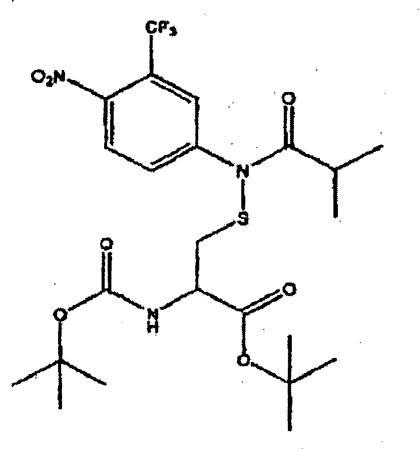 et des sels acceptables d'un point de vue pharmaceutique de celui-ci.

7. Un composé de la revendication 1 qui est : 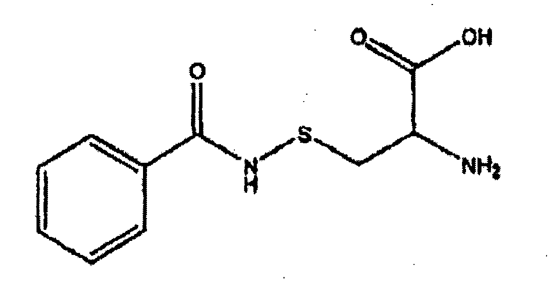 et des sels acceptables d'un point de vue pharmaceutique de celui-ci.

8. Un promédicament qui est un composé tel que défini dans la revendication 1 destiné à être utilisé en médecine.

9. Une composition pharmaceutique comprenant un promédicament tel que défini dans la revendication 8 et un support acceptable d'un point de vue pharmaceutique.

10. Une composition pharmaceutique telle que revendiquée dans la revendication 9 comprenant de plus un agent antimicrobien, un conservateur, un agent solubilisant, ou un agent stabilisant.

11. Un promédicament tel que revendiqué dans la revendication 8 qui est : 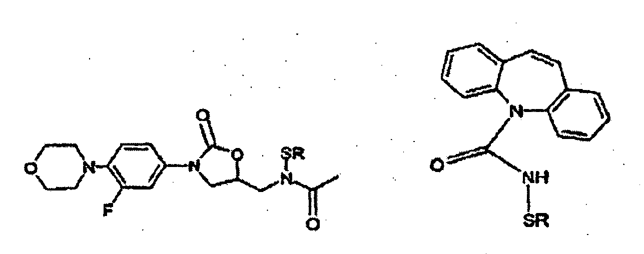 ou 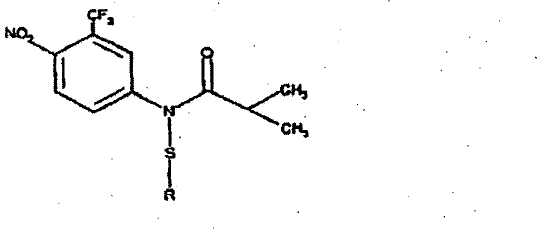 et des sels acceptables d'un point de vue pharmaceutique de celui-ci.

12. Une méthode pour faire un promédicament d'un composé pharmaceutique contenant une liaison N-H comprenant la réaction illustrée sur le schéma de réaction IV 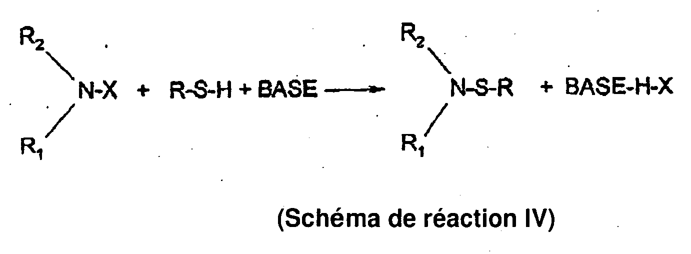
où X est n'importe quel bon groupe partant,
où R₁ et R₂ sont des résidus d'un composé pharmaceutique contenant une liaison N-H, R est tel que défini dans la revendication 1.

13. La méthode de la revendication 12 dans laquelle le composé pharmaceutique contenant une liaison N-H est un imide, une hydantoine, un uracile, un barbital, un amide, un benzamide, un N-méthylbenzamide, une carbamazépine, un flutamide, un linézolide, une urée, un sulfonamide, une oxazolidinone, ou un peptide ; ou un sel acceptable d'un point de vue pharmaceutique de ceux-ci.
